# EUROPÄISCHE PATENTANMELDUNG

(11) **EP 1 010 409 A1**
(43) Veröffentlichungstag der Anmeldung: **21.06.2000**
(21) Anmeldenummer: 99123243.0
(22) Anmeldetag: 26.11.1999
(51) Int. Cl.: A61F 5/01

(54) **Systemquengelgelenk für Knie und Knöchel**

(30) Priorität: 15.12.1998 DE 29822252 U
(71) Anmelder: Heinrich Caroli e.K., 77933 Lahr/Schwarzwald (DE)
(72) Erfinder: Müller, Horst, 77933 Lahr (DE)
(74) Vertreter: Richter, Werdermann & Gerbaulet

(57) **Zusammenfassung**

Ein Systemquengelgelenk besteht zur Verwendung an einem Knöchelgelenk oder einem Kniegelenk eines menschlichen Beines aus einem aus zwei Gelenkteilen (14, 16) gebildeten Gelenk (12) und aus diesen zugeordneten Ansatzschienen (10), wobei beidseits des Gelenkes (12) an den Gelenkteilen (14, 16) ein Befestigungsmittel (28, 30) zum lösbaren Befestigen einer jeweiligen Ansatzschiene (10) mit einem zugeordneten Gelenkteil (14, 16) vorgesehen sind.

## Beschreibung

Die Erfindung betrifft ein Systemquengelgelenk für Knie und Knöchel aus zwei Gelenkteilen.

Bekannt sind Quengel- und Gelenkschienen mit wenigstens einem aus zwei Gelenkteilen gebildeten Gelenk und jeweiligen, dem Gelenk zugeordneten Ansatzschienen. Derartige Schienen dienen mit vom Orthopäden eingesetzten Ansatzschienen zum Lösen von Kontrakturen (Gelenkversteifungen) oder auch zum Fixieren von Gelenken.

Systemquengelgelenke mit den Ausnehmungen in 12,6 mm und 20,0 mm Breite sind den gelenkübergreifenden Orthesenbau -von der Hüfte bis zum Knöchel- geeignet und können mit anderen Systemen kombiniert werden, da diese Einschübe für Ansatzschienen mit 12, 16 oder 20 mm Breite in der Orthopädie üblich sind.

Es ist Aufgabe der vorliegenden Erfindung, ein verbessertes Systemquengelgelenk für Knie und Knöchel zur Verfügung zu stellen.

Diese Aufgabe wird durch ein Systemquengelgelenk der o. g. Art mit den in Anspruch 1 gekennzeichneten Merkmalen gelöst.

Dazu ist es erfindungsgemäß vorgesehen, dass beidseits des Gelenkes an den Gelenkteilen ein Befestigungsmittel zum lösbaren Befestigen einer jeweiligen Ansatzschiene mit einem zugeordneten Gelenkteil vorgesehen ist.

Dies hat den Vorteil, dass ein Systemgelenk zur Verfügung steht, welches wahlweise mit unterschiedlichen Ansatzschienen, beispielsweise zur Verwendung an einem Knöchelgelenk oder einem Kniegelenk eines menschlichen Beines, verbindbar ist. Hierdurch sind mit geringer Lagerhaltung unterschiedliche Anwendungen an Gelenken mit unterschiedlichen Abmessungen durch einfaches kombinieren entsprechender Gelenke mit entsprechenden Ansatzschienen realisierbar.

Vorzugsweise Weitergestaltungen der Vorrichtung sind in den Ansprüchen 2 bis 8 beschrieben.

Für eine einfache und schnell herstellbare Verbindung zwischen Gelenk und Ansatzschiene umfasst das Befestigungsmittel an einem Gelenkteil wenigstens eine, insbesondere zwei Schrauben mit entsprechenden Gewindebohrungen, wobei an den zugehörigen Ansatzschienen mit den Gewindebohrungen fluchtende Öffnungen zum Hindurchgreifen der Schrauben ausgebildet sind.

Zum Sicherstellen einer drehfesten Verbindung zwischen einem Gelenkteil und einer zugeordneten Ansatzschiene weist jedes Gelenkteil im Bereich der Befestigungsmittel eine Ausnehmung auf, welche einer Kontur der diesem Gelenkteil zugeordneten Ansatzschiene entspricht.

Zum genauen Einstellen eines Winkels zwischen den mit einem Gelenk verbundenen Ansatzschienen umfasst das Gelenk ein Schneckengetriebe, welches eine Schnecke und ein Schneckenrad aufweist, wobei das Schneckenrad an einem ersten Gelenkteil angeordnet und die Schnecke an einem zweiten Gelenkteil drehbar und mit dem Schneckenrad in Eingriff stehend gehaltert ist.

Zweckmäßigerweise ist das Schneckenrad als Teilrad einstückig mit dem ersten Gelenkteil ausgebildet.

Zum Justieren eines Winkels zwischen zwei dem Gelenk zugeordneten Ansatzschienen mittels eines Gabelschlüssels oder einer Nuss weist die Schnecke eine aus dem zweiten Gelenkteil herausragenden Sechskant auf.

In einer bevorzugten Ausführungsform umfasst das zweite Gelenkteil zwei lösbar miteinander verbindbare Teile, welche zwischen sich die Schnecke haltern.

Zum seitlichen Versetzten eines Drehpunktes des Gelenkes, beispielsweise für Anwendungen an Kniegelenken, ist wenigsten ein Gelenkteil bzw. sind beide Gelenkteile eines Gelenkes zwischen dem Gelenk und dem Befestigungsmittel abgewinkelt.

Die Systemquengelgelenke werden Satzweise, jeweils zwei Stück, angebracht, nämlich lateral (aussen) und medial (innen), so daß sich die Drehpunkte genau gegenüberliegen. Die Quengelung wird synchron mit zwei Steckschlüsseln vorgenommen, um Verspannungen und Brücke zu vermeiden.

Nachstehend wird die Erfindung anhand der beigefügten Zeichnungen näher erläutert. Diese zeigen in
- Fig. 1: eine Ausführungsform eines Systemquengelgelenkes in perspektivischer Ansicht,
- Fig. 2: das Systemquengelgelenk von Fig. 1 in teilweiser Explosionsdarstellung und
- Fig. 3: eine weitere bevorzugte Ausführungsform eines Systemquengelgelenkes in Aufsicht.

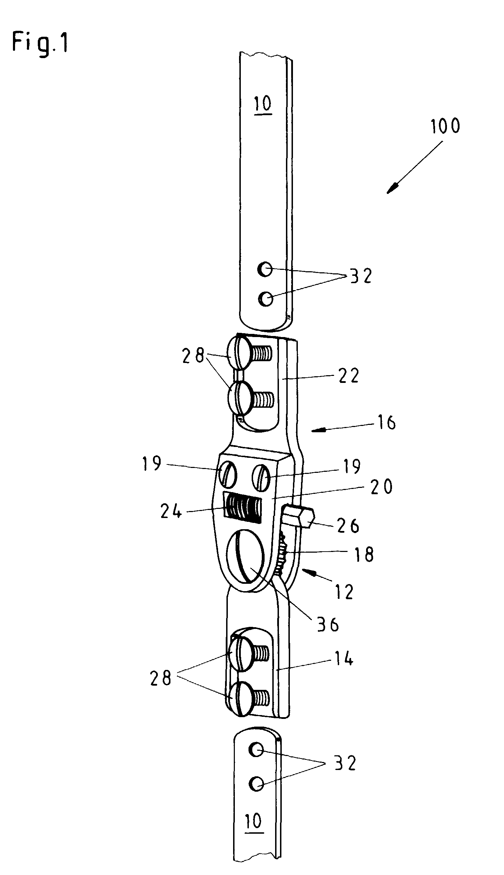

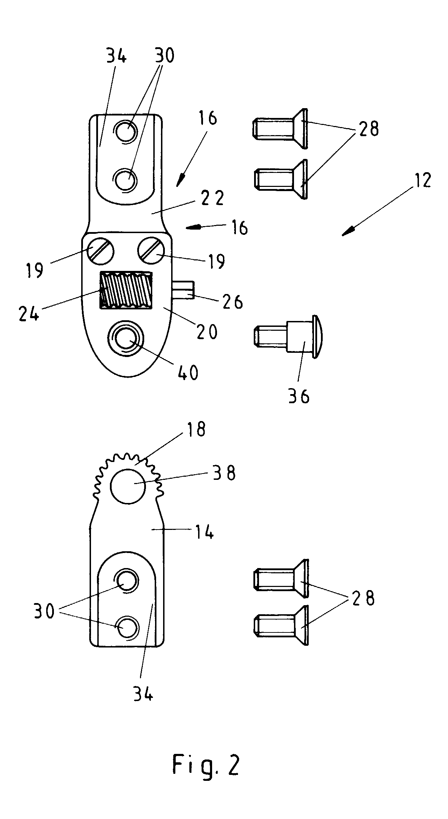

Die in Fig. 1 und 2 dargestellte bevorzugte Ausführungsform eines Systemquengelgelenkes umfasst zwei Ansatzschienen 10, welche über ein Gelenk 12 schwenkbar miteinander verbunden sind. Das Gelenk 12 ist aus einem ersten Gelenkteil 14 und einem zweiten Gelenkteil 16 gebildet. Am ersten Gelenkteil 14 ist einstückig mit diesem ein Schneckenrad 18 als Teilrad ausgebildet, während das zweite Gelenkteil 16 zwei mittels Schrauben 19 lösbar miteinander verbindbare Teile 20 und 22 umfasst, welche zwischen sich eine Schnecke 24 derart haltern, dass diese drehbar ist und mit dem zwischen den Teilen 20, 22 schwenkbar verschraubten Schneckenrad 18 in Eingriff steht.

Die Schnecke 24 weist einen über das zweite Gelenkteil 16 hinausragenden Sechskant 26 auf, wobei mittels Drehen am Sechskant 26 mit einem geeigneten Werkzeug, wie beispielsweise einem Gabelschlüssel oder einer Nuss, ein Schwenkwinkel zwischen den beiden Gelenkteilen 14, 16 und damit zwischen den an diesen lösbar befestigten Ansatzschienen 10 einstellbar ist.

Zum lösbaren Verbindung zwischen einer Ansatzschiene 10 und einem zugeordneten Gelenkteil 14 bzw. 16 sind am jedem Gelenkteil 14 bzw. 16 zwei Schrauben 28 mit zugehörigen Gewindebohrungen 30 vorgesehen. An der Ansatzschiene 10 sind zwei mit den Gewindebohrungen 30 fluchtende Öffnungen 32 zum Hindurchgreifen der Schrauben 28 vorgesehen.

Zur Montage der Ansatzschienen 10 werden diese in eine entsprechend der Kontur der Ansatzschiene am Gelenkteil 14 bzw. 16 ausgebildete Ausnehmung 34 angesetzt, wobei hier die Öffnungen 32 der Ansatzschiene 10 mit den Gewindebohrungen 30 des entsprechenden Gelenkteiles 14 bzw. 16 fluchten. Durch die Öffnungen 32 der Ansatzschiene 10 hindurch werden dann die Schrauben 28 in die Gewindebohrungen 30 des Gelenkteiles 14 bzw. 16 eingeschraubt und festgezogen. Hierdurch ist ein feste, jedoch lösbare Verbindung zwischen dem Gelenk 12 und den zugehörigen Ansatzschienen 10 hergestellt.

Durch eine entsprechende Ausbildung der Ausnehmung 34 bzw. Anordnung von Gewindebohrungen 30 und Öffnungen 32 wird beispielsweise sichergestellt, dass nur vorbestimmte Ansatzschienen an die Gelenkteile 14 bzw. 16 anschraubbar sind, so dass falsche Zuordnungen von Gelenken 12 zu Ansatzschienen 10 vermieden sind. Beispielsweise sind an ein Gelenk 12 für Kniegelenke eines menschlichen Beines nur Ansatzschienen 10 für Kniegelenke, jedoch keine Ansatzschienen 10 für Knöchelgelenke eines menschlichen Beines anschraubbar, da nur bei Ansatzschienen 10 für Kniegelenke die jeweiligen Öffnungen 32 dieser Ansatzschienen 10 mit den Gewindebohrungen 30 der Gelenkteile 14 bzw. 16 fluchten.

Die Gelenkteile sind mittels einer Schraube 36 an einem Punkt schwenkbar miteinander verbunden, wobei das erste Gelenkteil 14 zwischen die Teile 18, 20 des zweiten Gelenkteiles 16 greift und die Schraube 36 durch eine Öffnung 36 des ersten Gelenkteiles 14 hindurch in eine Gewindebohrung 40 des zweiten Gelenkteiles 16 greift. Die Schraube 36 bildet somit einen Drehpunkt für eine Schwenkbewegung zwischen den Gelenkteilen 14 und 16 und damit zwischen an den Gelenkteilen 14 bzw. 16 befestigten Ansatzschienen 10.

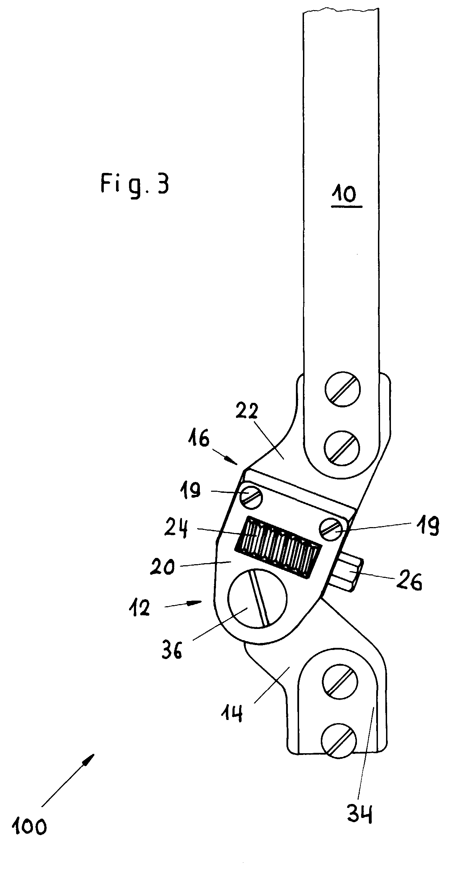

Fig. 3 zeigt eine alternative Ausführungsform 200 eines Systemquengelgelenkes mit einem anders ausgeführten Gelenk 12, welche vorwiegend an einem Kniegelenk eines menschlichen Beines zum Einsatz kommt. In Fig. 3 sind gleiche Teile mit gleichen Bezugsziffern wie bei der ersten Ausführungsform gemäß der Fig. 1 und 2 bezeichnet, so dass zu deren Erläuterung auf die obige Beschreibung der ersten Ausführungsform 100 und die Fig. 1 und 2 verwiesen wird.

Bei diesem Systemquengelgelenkes 200 sind die Gelenkteile 14 und 16 zwischen den Befestigungsmitteln 28, 30 und dem Gelenk 12 abgewinkelt ausgebildet, so dass der durch die Schraube 36 gebildete Drehpunkt bezüglich einer Längsachse der Ansatzschiene 10 seitlich versetzt ist. Fig, 3 veranschaulicht ferner eine am zweiten Gelenkteil 16 festgeschraubte Ansatzschiene 10.

## Patentansprüche

1. Systemquengelgelenk (100, 200) mit wenigstens einem aus zwei Gelenkteilen (14, 16) gebildeten Gelenk (12) und jeweiligen, dem Gelenk (12) zugeordneten Ansatzschienen (10), wobei beidseits des Gelenkes (12) an den Gelenkteilen (14, 16) ein Befestigungsmittel (28, 30) zum lösbaren Befestigen einer jeweiligen Ansatzschiene (10) mit einem zugeordneten Gelenkteil (14, 16) vorgesehen sind.

2. Systemquengelgelenk (100, 200) nach Anspruch 1,
dadurch gekennzeichnet,
dass das Befestigungsmittel an einem Gelenkteil (14, 16) wenigstens eine, insbesondere zwei Schrauben (28) mit entsprechenden Gewindebohrungen (30) umfasst, wobei an den zugehörigen Ansatzschienen (10) mit den Gewindebohrungen (30) fluchtende Öffnungen (32) zum Hindurchgreifen der Schrauben (28) ausgebildet sind.

3. Systemquengelgelenk (100, 200) nach Anspruch 1 oder 2,
dadurch gekennzeichnet,
dass jedes Gelenkteil (14, 16) im Bereich der Befestigungsmittel (28, 30) eine Ausnehmung (34) aufweist, welche einer Kontur der diesem Gelenkteil (14, 16) zugeordneten Ansatzschiene (10) entspricht.

4. Systemquengelgelenk (100, 200) nach einem der Ansprüche 1 bis 3,
dadurch gekennzeichnet,
dass das Gelenk (10) ein Schneckengetriebe umfasst, welches eine Schnecke (24) und ein Schneckenrad (18) aufweist, wobei das Schneckenrad (18) an einem ersten Gelenkteil (14) angeordnet und die Schnecke (24) an einem zweiten Gelenkteil (16) drehbar und mit dem Schneckenrad (18) in Eingriff stehend gehaltert ist.

5. Systemquengelgelenk (100, 200) nach Anspruch 4,
dadurch gekennzeichnet,
dass das Schneckenrad (18) als Teilrad einstückig mit dem ersten Gelenkteil (14) ausgebildet ist.

6. Systemquengelgelenk (100, 200) nach Anspruch 4 oder 5,
dadurch gekennzeichnet,
dass die Schnecke (14) eine aus dem zweiten Gelenkteil (16) herausragenden Sechskant (26) aufweist.

7. Systemquengelgelenk (100, 200) nach einem der Ansprüche 4 bis 6,
dadurch gekennzeichnet,
dass das zweite Gelenkteil (16) zwei lösbar miteinander verbindbare Teile (20, 22) umfasst, welche zwischen sich die Schnecke (24) haltern.

8. Systemquengelgelenk (200) nach einem der Ansprüche 1 bis 7,
dadurch gekennzeichnet,
dass wenigsten ein Gelenkteil (14, 16), insbesondere beide Gelenkteile (14, 16), eines Gelenkes (12) zwischen dem Gelenk (12) und dem Befestigungsmittel (28, 30) abgewinkelt ist bzw. sind.
